(19) Europäisches Patentamt
European Patent Office
Office européen des brevets

(11) Veröffentlichungsnummer: **0 273 409**
**A1**

# (12) EUROPÄISCHE PATENTANMELDUNG

(21) Anmeldenummer: **87119194.6**

(22) Anmeldetag: **24.12.87**

(51) Int. Cl.[4]: **C07C 79/35** , C07C 79/46 , C07C 121/75 , C07C 76/02 , C07C 43/225 , C07C 41/00 , C07C 69/92 , A01N 43/40 , A01N 43/54

(30) Priorität: **31.12.86 DE 3644798**

(43) Veröffentlichungstag der Anmeldung: **06.07.88 Patentblatt 88/27**

(84) Benannte Vertragsstaaten: **AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Fuss, Andreas, Dr.**
**Lindigstrasse 24**
**D-8757 Karlstein(DE)**
Erfinder: **Giencke, Wolfgang, Dr.**
**Am Steinberg 45**
**D-6238 Hofheim am Taunus(DE)**
Erfinder: **Siegemund, Günter, Dr.**
**Frankfurter Strasse 21**
**D-6238 Hofheim am Taunus(DE)**

## (54) Neue Nitrohaloalkoxybenzole, Verfahren zu ihrer Herstellung und ihre Verwendung.

(57) Nitrohaloalkoxybenzole der Formel I

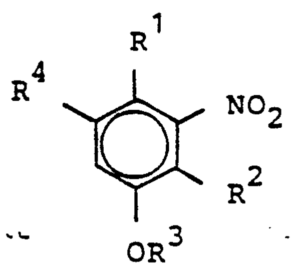

(I),

worin
$R^1$ OH, Halogen,
$R^2$ H, Halogen,
$R^3$ Fluor enthaltendes, wenigstens teilweise halogeniertes ($C_1$-$C_3$)-Alkyl, wobei in teilweise halogenierten Resten die Zahl der Halogenatome die von Wasserstoff übersteigt und $R^3$ dann, wenn $R^1$ OH ist, höchstens ein von Fluor verschiedenes Halogenatom enthält,
$R^4$ Nitro, Cyano, Trihalomethyl oder Alkoxycarbonyl, worin der Alkoxyrest 1 bis 3 C-Atome hat, bedeutet, mit der Maßgabe, daß $R^4$ nicht Nitro bedeutet, wenn $R^1$ OH und $R^3$ $CF_2$-$CHCl_2$ ist.
Die Erfindung bezieht sich weiter auf ein Verfahren zur Herstellung solcher Verbindungen der Formel II

EP 0 273 409 A1

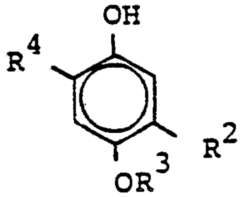

(II)

worin die Reste $R^2$, $R^3$ und $R^4$ dieselbe Bedeutung besitzen wie in der Formel I und $R^4$ zusätzlich H sein kann, und auf Verbindungen der Formel VI

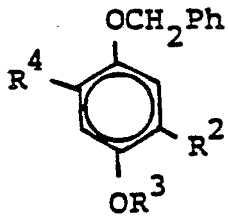

(VI)

wobei Ph Phenyl und $R^2$, $R^3$ und $R^4$ dieselbe Bedeutung haben wie in Formel II mit der Maßgabe, daß der Rest $R^3$ mindestens 2 C-Atome enthält.

## Neue Nitrohaloalkoxybenzole, Verfahren zu ihrer Herstellung und ihre Verwendung

Aus der US-PS 3 781 369 sind substituierte Haloalkoxy-und Haloalkylthio-phenol-oder thiophenolverbindungen der Formel

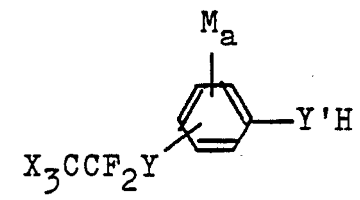

bekannt, worin jedes X unabhängig voneinander Wasserstoff, Brom, Chlor oder Fluor darstellt mit der Maßgabe, daß mindestens ein X Brom, Chlor oder Fluor ist, Y und Y' jeweils unabhängig voneinander Sauerstoff oder Schwefel, M jeweils unabhängig voneinander Brom, Chlor, Fluor, Jod, Nitro oder niederes Alkyl mit 1 bis 4 C-Atomen und a eine ganze Zahl von 1 bis 3 darstellt. Diese Verbindungen sind als Herbizide, Fungizide und Insektizide sowie als Zwischenprodukte für die Herstellung von substituierten Äthylphenoxyacetaten geeignet. Unter den zahlreichen dort beschriebenen Verbindungen sind auch einige Mono-, Di-und Trinitroverbindungen. Als einziges 2,6-Dinitrophenolderivat, das in 4-Stellung die Gruppe $X_3CCF_2Y$ trägt, wird im Beispiel 10 das 4-(2,2-Dichlor-1,1-difluoräthoxy)-2,6-dinitrophenol vom Schmelzpunkt 31-53°C beschrieben. Als einziges weiteres 2,6-Dinitrophenol wird im Beispiel 40 das 4-Chlor-3-(2,2-dichlor-1,1-difluoräthoxy)-2,6-dinitrophenol beschrieben, das also in 4-Stellung ein Chloratom und die Dichlordifluoräthoxygruppe in 3-Stellung enthält. Abgesehen von dem 4-(2,2-dichlor-1,1-difluoräthoxy)-2-nitrophenol (Beispiel 9) werden im übrigen nur solche Mono-, Di-und Trinitroverbindungen beschrieben, in denen die $X_3CCF_2Y$ Gruppe sich in einer anderen Position als der 4-Stellung befindet.

Gegenstand der vorliegenden Erfindung sind nun neue Verbindungen der Formel I

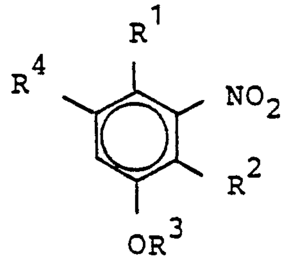

(I),

worin
$R^1$ OH, Halogen,
$R^2$ H, Halogen,
$R^3$ Fluor enthaltendes, wenigstens teilweise halogeniertes $(C_1\text{-}C_3)$-Alkyl, wobei in teilweise halogenierten Resten die Zahl der Halogenatome die von Wasserstoff übersteigt und $R^3$ dann, wenn $R^1$ OH ist, höchstens ein von Fluor verschiedenes Halogenatom enthält,
$R^4$ Nitro, Cyano, Trihalomethyl oder Alkoxycarbonyl, worin der Alkoxyrest 1 bis 3 C-Atome hat, bedeutet, mit der Maßgabe, daß $R^4$ nicht Nitro bedeutet, wenn $R^1$ OH und $R^3$ $CF_2\text{-}CHCl_2$ ist.

Bevorzugt sind Verbindungen, in denen $R^4$ dann nicht Nitro bedeutet, wenn $R^1$ OH und $R^3$ $CF_2\text{-}CHX_2$ ist, worin mindestens ein X fluor, Chlor oder Brom ist und das andere auch Wasserstoff sein kann.

In den erfindungsgemäßen Verbindungen ist unter Halogen in den Resten $R^1$ bis $R^4$ unabhängig voneinander jeweils F, Cl, Br oder Jod, insbesondere F, Cl, Br zu verstehen. Das halogenierte Alkyl in $R^3$ enthält mindestens 1, vorzugsweise mindestens 2 Fluoratome und ist z.B. $CHF_2$, $CFCl_2$, $CF_2Cl$, $CF_3$, $CF_2CHF_2$, $CF_2CHCl_2$, $CF_2CHFCl$, $CF_2CHFBr$, $CH_2CF_3$, $CF_2CF_3$ oder $CF_2CHFCF_3$, insbesondere $CHF_2$, $CF_3$, $CF_2CHF_2$, $CF_2CHFCl$, $CF_2CHFCF_3$. Bevorzugt sind also mindestens 2 Fluoratome an dem dem Sauerstoffatom benachbarten C-Atom gebunden.

In $R^4$ kann die Trihalomethylgruppe gleiche oder verschiedene Halogenatome, insbesondere Fluor, Chlor und/oder Brom enthalten. Bevorzugt ist sie die Trifluormethylgruppe. In dem Alkoxycarbonylrest kann

der Alkoxyrest z.B. Methoxy, Äthoxy, Propoxy oder Isopropoxy sein. Bevorzugt sind die Verbindungen der Formel I, worin $R^4$ Nitro bedeutet.

Gegenstand der vorliegenden Erfindung ist auch ein Verfahren zur Herstellung der Verbindungen der Formel I, das dadurch gekennzeichnet ist, daß man

a)Verbindungen der Formel II

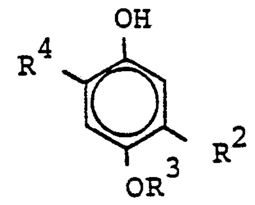

(II),

worin die Reste $R^2$, $R^3$ und $R^4$ dieselbe Bedeutung besitzen wie in der Formel I und $R^4$ zusätzlich H sein kann, unter üblichen Bedingungen zu Verbindungen der Formel I mit $R^1$ = OH nitriert und die erhaltenen Substanzen I isoliert oder zu Verbindungen der Formel I mit $R^1$ = Halogen halogeniert.

Die als Ausgangsmaterialien in Verfahren a) verwendeten Verbindungen der Formel II und ihre Herstellung sind bekannt. Diese Verbindungen und solche, in denen nebeneinander $R^1$ OH, $R^3$ $CF_2$-$CHX_2$ - (worin mindestens ein X Fluor, Chlor oder Brom ist und das andere auch Wasserstoff sein kann) und $R^4$ Nitro bedeutet, lassen sich auch nach einem neuen Verfahren herstellen, das ebenfalls Gegenstand der Erfindung ist. Danach setzt man

b) Verbindungen der Formel III

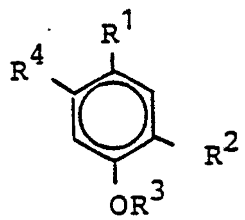

(III),

worin
$R^1$ Fluor,
$R^2$ Chlor, Brom, Jod oder Wasserstoff bedeuten und $R^3$ und $R^4$ dieselbe Bedeutung besitzen wie in Formel I, $R^4$ aber auch H sein kann,
mit einem Alkali-bzw. Erdalkalihydroxid zu Verbindungen der Formel II um und isoliert diese oder setzt sie gemäß a) weiter um.

Verbindungen der Formel II, in denen $R^3$ die unten angegebenen Bedeutungen hat, lassen sich jedoch nicht nur nach dem Verfahren der US-PS 3 781 369, sondern auch nach einem weiteren neuen Verfahren herstellen, welches ebenfalls Gegenstand der Erfindung ist. Danach lagert man

c) ein Phenol der Formel IV

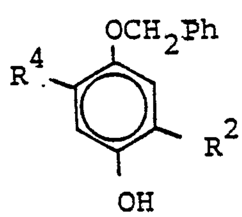

(IV),

in dem
$R^2$ und $R^4$ dieselbe Bedeutung haben wie in Formel II

$C_1$) an eine halogeniertes Alken der Formel $CF_2=CX_2$ (V), worin mindestens ein X Halogen, vorzugsweise F, Cl und/oder Br, und ein X auch $CF_3$ oder H bedeuten kann, an und stellt so Verbindungen her, in denen $R^3$ den Rest $-CF_2-CHX_2$ bedeutet, oder setzt

$C_2$) mit Trifluormethansulfonsäure-2,2,2-trifluoräthylester in Gegenwart einer Base zu Verbindungen um, in denen $R^3$ den Rest $-CH_2-CF_3$ darstellt,

und debenzyliert die erhaltenen Verbindungen der Formel VI

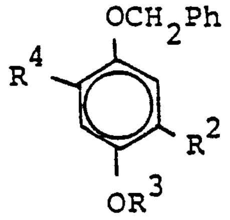

(VI),

in denen $R^2$, $R^3$ und $R^4$ die unter c) angegebene Bedeutung haben, hydrogenolytisch oder durch Behandlung mit Mineralsäure zu Verbindungen der Formel II. Dieses Verfahren c) liefert höhere Ausbeuten an Verbindungen der Formel II als die bekannten Verfahren.

Die Verbindungen der Formel VI, in den $R^3$ mindesten zwei C-Atome enthält, sind neu und ebenfalls Gegenstand der Erfindung.

Die Nitrierung gemäß a) wird z.B. in der Weise durchgeführt, daß das Phenol der Formel II zu Salpetersäure zudosiert und das Gemisch bei einer Temperatur von im allgemeinen 0 bis 75°C, vorzugsweise 0 bis 30°C gehalten wird. Wenn die Hauptreaktion abgeklungen ist, kann die Reaktion durch Erhöhen der Temperatur zu Ende geführt und so eine vollständige Umsetzung erzielt werden. Gegebenenfalls kann die Nitrierung auch in Gegenwart eines gegenüber den Reaktionsteilnehmern inerten Lösungsmittels und/oder unter Zusatz einer Mineralsäure durchgeführt werden. Der angewandte Druck ist bei allen Ausführungsformen nicht kritisch, und die Reaktion wird normalerweise unter gewöhnlichem Druck durchgeführt. Die Konzentration der Salpetersäure beträgt im allgemeinen 10 bis 100, vorzugsweise 65 bis 100 Gew.-%. Als Lösungsmittel können z.B. Dichlormethan, Chloroform, 1,2-Dichloräthan, Tetrachlormethan, Nitromethan, Dimethylsulfoxid oder Sulfolan eingesetzt werden. Als Mineralsäure können z.B. Schwefelsäure oder Flußsäure verwendet werden. Ihr Anteil beträgt bis 100 Gew.-%, bezogen auf die Salpetersäure, vorzugsweise 20 bis 80 Gew.-%. Die Temperaturen liegen bei dieser Ausführungsform im allgemeinen zwischen -2 und +160°C, vorzugsweise zwischen 0 und 100°C. Nach einer anderen Ausführungsform kann die Nitrierung auch mit einem Metallnitrat wie Lithium-, Natrium-, Kalium-, Kupfer-, oder Magnesiumnitrat in Gegenwart einer Säure wie Schwefelsäure, Flußsäure, Essigsäure oder Trifluoressigsäure, zweckmäßig bei Temperaturen zwischen +50 und + 200°C, vorzugsweise zwischen +75 und +160°C durchgeführt werden.

Die Halogenierung gemäß a) wird in üblicher Weise durchgeführt. Zum Beispiel wird das Phenol der Formel I mit $R^1$ = OH mit einem Halogenierungsreagenz wie Thionylchlorid, Phosphoroxychlorid, Phosphorpentachlorid, Phenylphosphonsäuredichlorid, Phosphortribromid oder Schwefeltetrafluorid bei Temperaturen von 0°C bis +180°C in Gegenwart von 0 bis 100 mol-% Dimethylformamid, N,N-Dimethylanilin, Pyridin oder 4-Dimethylaminopyridin, gegebenenfalls in einem gegenüber den Reaktionsteilnehmern inerten Lösungsmittel wie Dichlormethan, Trichlormethan, Tetrachlormethan, Toluol oder Xylol umgesetzt.

Verbindungen der Formel II werden gemäß b) z.B. in der Weise erhalten, daß die Verbindungen der Formel III in einem gegenüber den Reaktionsteilnehmern inerten Lösungsmittel wie Dimethylformamid, Dimethylacetamid, N-Methylpyrrolidon, Dimethylsulfoxid, Dimethylsulfon oder Sulfolan bei Temperaturen zwischen +20 und +200°C, vorzugsweise bei 100 bis 180°C mit Alkali-bzw. Erdalkalihydroxiden (was quartäre Ammoniumhydroxide einschließen soll) wie Lithium-, Magnesium-, Natrium, Kalium-, Tetramethylammonium-, Barium-oder Calciumhydroxid umgesetzt werden. Der Anteil an Alkali beträgt im allgemeinen 100 bis 500 mol-%, bezogen auf die eingesetzte Verbindung der Formel III.

Verbindungen der Formel VI werden gemäß $c_1$) bzw. $c_2$) z.B. in der Weise erhalten, daß man das Phenol der Formel IV mit einem halogenierten Alken der Formel V wie Tetrafluoräthen, Trifluoräthen, Chlortrifluoräthen, Bromtrifluoräthen, Jodtrifluoräthen, 1,1-Dichlordifluoräthen, Hexafluorpropen oder mit Trifluormethansulfonsäure-2,2,2-trifluoräthylester, wie es bei E.H. Banitt, W.E. Coyne et al., J. Med. Chem. 18, 1130 (1975) beschrieben ist, in Gegenwart einer Base wie Alkalihydroxid, Alkalialkoholat, Alkalihydrid, Alkalicarbonat oder Alkaliamid in einem dipolar aprotischen Lösungsmittel wie Tetrahydrofuran, 1,4-Dioxan, Dimethoxyäthan, Acetonitril, Propionitril, Benzonitril, Dimethylformamid, Dimethylacetamid, N-Methylpyrrolidon, Dimethylsulfoxid, Sulfolan, Aceton oder Hexamethylphosphorsäuretriamid, im allgemeinen bei Temperaturen von -100 bis +250°C, vorzugsweise -10 bis +100°C, umsetzt. Der Anteil an eingesetzter Base beträgt zweckmäßig 0,1 bis 100 mol-%, bezogen auf eingesetztes Phenol der Formel IV.

Bei der Abspaltung der Benzylgruppe aus Verbindungen der Formel VI, die nach $C_1$) und $c_2$) erhalten

wurden, können als Mineralsäuren z.B. Fluorwasserstoff, HCl, HBr oder HJ, die jeweils 0 bis 200 Gew.-% Wasser enthalten können, eingesetzt werden. Zweckmäßig arbeitet man bei Temperaturen von -30 bis +150°C. Die Hydrogenolyse der Verbindungen VI wird zweckmäßig analog E. Reimann (Houben-Weyl, Band 4/1c, Seite 385 ff (1980), G. Thieme Verlag, Stuttgart und dort zitierte Literatur) mit Palladium auf Aktivkohle als Katalysator mit Wasserstoffgas bei Drucken bis 100 bar und Temperaturen von 0 bis 75°C in Lösungsmitteln wie Alkoholen, Äthern oder Essigester durchgeführt.

Die Verbindungen der Formel I stellen wertvolle Zwischenprodukte für die Herstellung von Pflanzenschutzmitteln dar (siehe Deutsche Patentanmeldung P 36 18 815.8 (HOE 86/F 129) für neue Pyrimidin-Derivate, deren Herstellung und Verwendung sowie europäische Patentschrift 31257 für Pyridylaniline). Ferner besitzen die Verbindungen der Formel I teilweise selbst herbizide, insektizide oder fungizide Eigenschaften.

Die Erfindung wird durch nachfolgende Beispiele erläutert. Darin bedeutet Schmp. Schmelzpunkt und Sdp Siedepunkt.

Beispiele für neue Stoffe

1) 2,6-Dinitro-4-(1,1,2,2-tetrafluoräthoxy)-phenol 42 g 4-(1,1,2,2-Tetrafluoräthoxy)-phenol wurden bei 10°C in 63 g 65-prozentiger Salpetersäure unter Rühren eingetragen und 1h bei 75°C gerührt. Man kühlte mit Eis, saugte den gelben Feststoff ab und wusch 2 Mal mit je 50 ml Wasser nach. Nach Trocknung unter vermindertem Druck erhielt man 38 g gelbe Kristalle vom Schmp. 82 - 83°C.

2) 2,6-Dinitro-4-(1,1,2,2-tetrafluoräthoxy)-chlorbenzol Zu einer Mischung aus 15 g 2,6-Dinitro-4-(1,1,2,2-tetrafluoräthoxy)-phenol, 2 ml Dimethylformamid und 100 ml trockenem Tetrachlormethan wurden 15 ml Thionylchlorid bei 20-25°C unter Rühren zugetropft und die Mischung 18h zum Sieden erhitzt. Man fitrierte heiß und dampfte das Lösungsmittel unter vermindertem Druck ein. Eine Destillation ergab 13 g einer gelben Flüssigkeit vom Sdp. 120 - 126°C bei 0,15 bis 0,2 mbar.

Beispiele für neue Verfahren

3) 4-(Trifluormethoxy)-phenol (Verfahren b) Eine Mischung aus 1,8 g 4-(Trifluormethoxy)-fluorbenzol, 2,24 KOH-Pulver und 10 ml trockenem Dimethylsulfoxid wurde 3h bei 150°C gerührt. Nach dem Abkühlen versetzte man mit 10 ml 33-prozentiger NaOH und 50 ml $H_2O$ und extrahierte zweimal mit je 100 ml Dichlormethan. Man säuerte die wäßrige Phase mit konz. HCl an und extrahierte zweimal mit je 50 ml Diäthyläther. Man trocknete über $Na_2SO_4$, dampfte den Äther unter vermindertem Druck ab und destillierte den Rückstand. Man erhielt 1,2 g einer farblosen Flüssigkeit vom Sdp. 90°C bei 4,5 mbar. (W.A. Sheppard, J.Org.Chem. 29,1 (1964) geben den Sdp. mit 92°C/25 Torr an).

4) 4-Benzyloxy-(1,1,2,2-tetrafluoräthoxy)-benzol (Verfahren $c_1$) In einem 1 l Rührautoklaven aus Chromnickelstahl wurde 8h bei 40°C und 7,5 bar Tetrafluoräthen auf eine Mischung aus 100 g 4-Benzyloxyphenol, 69,1 g Kaliumcarbonat und 0,5 l trockenem Acetonitril unter Rühren aufgepreßt. Das Produkt wurde mit 500 ml Chloroform aufgenommen, viermal mit je 100 ml $H_2O$ gewaschen, über $Na_2SO_4$ getrocknet und das Lösungsmittel am Rotationsverdampfer eingedampft. Der Rückstand wurde aus Hexan umkristallisiert. Man erhielt 144,6 g farblose Kristalle vom Schmp. 62-64°C.

5) 4-(1,1,2,2-Tetrafluoräthoxy)-phenol (Verfahren c) In einem 5 l-Autoklaven aus Chromnickelstahl wurden 773 g 4-Benzyloxy-(1,1,2,2-tetrafluoräthoxy)-benzol in 3 l Essigester und 40 g 10 % Palladium auf Aktivkohle unter Rühren bei 20-25°C mit 50 bar Wasserstoffdruck hydriert, bis keine Aufnahme mehr beobachtet wurde (8h). Man filtrierte über ein Filtrierhilfsmittel ((®)Celite), dampfte das Lösungsmittel unter vermindertem Druck ein und führte mit dem Rückstand eine Feststoffdestillation durch. Man erhielt 497 g farblose Kristalle vom Schmp. 46,5-47,5°C und Sdp. 74°C/0,1 mbar (DOS 32 13 152 Sdp. 80°C/1,5 mbar)

Tabelle 1
(Vorprodukte)

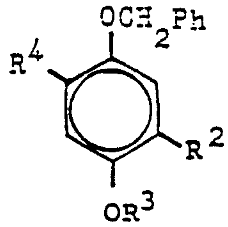

(VI)

| Nr. | $R^2$ | $R^3$ | $R^4$ | Schmp. (°C) | Sdp. (°C / mbar) |
|---|---|---|---|---|---|
| 6 | H | $CHF_2$ | H | 50-53 | 119/0.02 |
| 7 | H | $CF_2CHFCl$ | H | 61-62 | |
| 8 | H | $CF_2CHFBr$ | H | 59-61 | |
| 9 | H | $CF_2CHCl_2$ | H | 77 | |
| 10 | H | $CH_2CF_3$ | H | | |
| 11 | H | $CF_2CHFCF_3$ | H | 65 | |

Tabelle 2

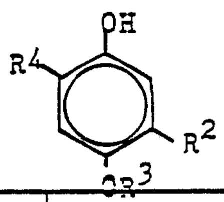

(II)

| Nr. | $R^2$ | $R^3$ | $R^4$ | Schmp. (°C) | Sdp. (°C / mbar) |
|---|---|---|---|---|---|
| 12 | H | $CHF_2$ | H | Öl | 61-63/0.1 |
| 13 | H | $CF_2Cl$ | H | | |
| 14 | H | $CF_2CHFCl$ | H | 43-44 | |
| 15 | Cl | $CF_2CHFCl$ | H | | |
| 16 | H | $CF_2CHFCl$ | $CF_3$ | | |
| 17 | H | $CF_2CHFCl$ | CN | | |
| 18 | H | $CF_2CHFCl$ | $COOCH_3$ | | |
| 19 | H | $CF_2CHFBr$ | H | 42-44 | 86/0.15 |
| 20 | H | $CF_2CHCl_2$ | H | 49-51 | 80/0.15 |
| 21 | H | $CH_2CF_3$ | H | | |
| 22 | H | $CF_2CHFCF_3$ | H | | 51/0.1 |

Tabelle 3 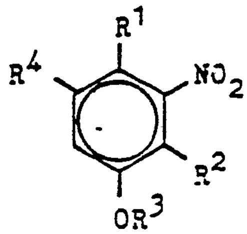 (I)

| Nr. | $R^1$ | $R^2$ | $R^3$ | $R^4$ | Schmp. (°C) | Sdp. (°C / mbar) |
|---|---|---|---|---|---|---|
| 23 | OH | H | $CHF_2$ | $NO_2$ | | 149-151/0.3 |
| 24 | OH | H | $CF_3$ | $NO_2$ | 33-34 | |
| 25 | OH | H | $CF_2Cl$ | $NO_2$ | | |
| 26 | OH | H | $CF_2CHFCl$ | $NO_2$ | 77-78 | |
| 27 | OH | Cl | $CF_2CHFCl$ | $NO_2$ | | |
| 28 | OH | H | $CF_2CHFCl$ | CN | | |
| 29 | OH | H | $CF_2CHFCl$ | $CF_3$ | | |
| 30 | OH | H | $CF_2CHFCl$ | $COOCH_3$ | | |
| 31 | OH | H | $CF_2CHFBr$ | $NO_2$ | | 122-124/0.15 |
| 32 | OH | H | $CF_2CHCl_2$ | $NO_2$ | 51-53 | |
| 33 | OH | H | $CH_2CF_3$ | $NO_2$ | | |
| 34 | OH | H | $CF_2CHFCF_3$ | $NO_2$ | 57-59 | |
| 35 | Cl | H | $CHF_2$ | $NO_2$ | | |
| 36 | Cl | H | $CF_3$ | $NO_2$ | 44-45 | |
| 37 | F | H | $CF_3$ | $NO_2$ | | |
| 38 | Cl | H | $CF_2Cl$ | $NO_2$ | | |
| 39 | Cl | H | $CF_2CHFCl$ | $NO_2$ | | 144-6/0.2 |
| 40 | Cl | Cl | $CF_2CHFCl$ | $NO_2$ | | |
| 41 | Cl | H | $CF_2CHFCl$ | CN | | |
| 42 | Cl | H | $CF_2CHFCl$ | $CF_3$ | | |
| 43 | Cl | H | $CF_2CHFCl$ | $COOCH_3$ | | |
| 44 | Cl | H | $CF_2CHFBr$ | $NO_2$ | | |
| 45 | Cl | H | $CF_2CHCl_2$ | $NO_2$ | | 146/0.15 |
| 46 | Cl | H | $CH_2CF_3$ | $NO_2$ | | |
| 47 | Cl | H | $CF_2CHFCF_3$ | $NO_2$ | | 120-2/0.5 |

**Ansprüche**

1. Nitrohaloalkoxybenzole der Formel I

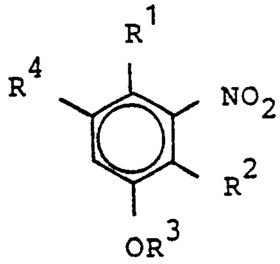

(I),

worin
$R^1$ OH, Halogen,
$R^2$ H, Halogen,
$R^3$ Fluor enthaltendes, wenigstens teilweise halogeniertes $(C_1-C_3)$-Alkyl, wobei in teilweise halogenierten Resten die Zahl der Halogenatome die von Wasserstoff übersteigt und $R^3$ dann, wenn $R^1$ OH ist, höchstens ein von Fluor verschiedenes Halogenatom enthält,
$R^4$ Nitro, Cyano, Trihalomethyl oder Alkoxycarbonyl, worin der Alkoxyrest 1 bis 3 C-Atome hat, bedeutet, mit der Maßgabe, daß $R^4$ nicht Nitro bedeutet, wenn $R^1$ OH und $R^3$ $CF_2$-$CHCl_2$ ist.

2. Verbindungen nach Anspruch 1, in denen $R^4$ dann nicht Nitro bedeutet, wenn $R^1$ OH und $R^3$ $CF_2$-$CHX_2$ ist, worin mindestens ein X fluor, Chlor oder Brom ist und das andere auch Wasserstoff sein kann.

3. Verbindungen nach Anspruch 1 oder 2, dadurch gekennzeichnet, daß das Halogen in den Resten $R^1$ bis $R^4$ F, Cl oder Br ist.

4. Verbindungen nach einem oder mehreren der Ansprüche 1 bis 3, dadurch gekennzeichnet, daß das halogenierte Alkyl in $R^3$ mindestens 1, insbesondere mindestens 2 Fluoratome enthält, wobei vorzugsweise mindestens 2 Fluoratome an dem dem Sauerstoffatom benachbarten C-Atom gebunden sind.

5. Verbindungen nach einem oder mehreren der Ansprüche 1 bis 4, dadurch gekennzeichnet, daß in $R^4$ die Trihalomethylgruppe die Trifluormethylgruppe und die Alkoxycarbonylgruppe die Methoxycarbonylgruppe ist, daß $R^4$ aber vorzugsweise Nitro ist.

6. Verfahren zur Herstellung von Verbindungen der Formel I, wie in einem oder mehreren der Ansprüche 1 bis 5 definiert, dadurch gekennzeichnet, daß man Verbindungen der Formel II

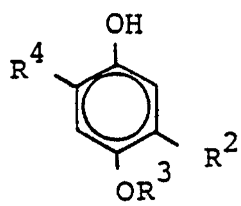

(II)

worin die Reste $R^2$, $R^3$ und $R^4$ dieselbe Bedeutung besitzen wie in der Formal I und $R^4$ zusätzlich H sein kann, zu Verbindungen der Formel I mit $R^1$ = OH nitriert und die erhaltenen Substanzen I isoliert oder zu Verbindungen der Formel I mit $R^1$ = Halogen halogeniert.

7. Verfahren zur Herstellung von Verbindungen der Formel II, worin
$R^2$ H, Halogen,
$R^3$ Fluor enthaltendes, wenigstens teilweise halogeniertes $(C_1-C_3)$-Alkyl, wobei in teilweise halogenierten Resten die Zahl der Halogenatome die von Wasserstoff übersteigt und $R^3$ dann, wenn $R^1$ OH ist, höchstens ein von Fluor verschiedenes Halogenatom enthält,
$R^4$ Nitro, Cyano, Trihalomethyl oder Alkoxycarbonyl, worin der Alkoxyrest 1 bis 3 C-Atome hat, bedeutet, dadurch gekennzeichnet, daß man Verbindungen der Formel III,

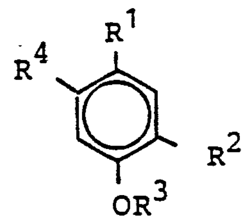

(III),

worin
$R^1$ Fluor,
$R^2$ Chlor, Brom, Jod oder Wasserstoff,
$R^3$ Fluor enthaltendes, wenigstens teilweise halogeniertes ($C_1$-$C_3$)-Alkyl, wobei in teilweise halogenierten Resten die Zahl der Halogenatome die von Wasserstoff übersteigt und $R^3$ dann, wenn $R^1$ OH ist, höchstens ein von Fluor verschiedenes Halogenatom enthält,
$R^4$ Nitro, Trihalomethyl oder Alkoxycarbonyl, worin der Alkoxyrest 1 bis 3 C-Atome hat, oder Wasserstoff beduetet, mit einem Alkali-bzw. Erdalkalihydroxyd zu Verbindungen der Formel II umsetzt und diese isoliert oder gemäß a) weiter umsetzt.

8. Verfahren zur Herstellung von Verbindungen der Formel II, wie im Anspruch 7 definiert, dadurch gekennzeichnet, daß man ein Phenol der Formel IV,

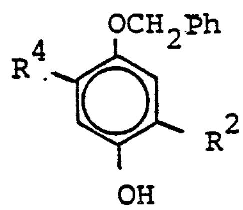

(IV)

in dem $R^2$ und $R^4$ dieselbe Bedeutung haben wie in Formel II, $c_1$) an ein halogeniertes Alken der Formel $CF_2=CX_2$ (V), worin mindestens ein X Halogen, vorzugsweise F, Cl und/oder Br, und eine X auch $CF_3$ oder H bedeuten kann, anlagert und so Verbindungen herstellt, in denen $R^3$ den Rest $-CF_2-CHX_2$ bedeutet, oder $C_2$) mit Trifluormethansulfonsäure-2,2,2-trifluoräthylester in Gegenwart einer Base zu Verbindungen umsetzt, in denen $R^3$ den Rest $-CH_2-CF_3$ darstellt,
und die erhaltenen Verbindungen der Formel VI,

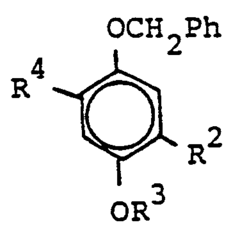

(VI)

in der $R^2$, $R^3$ und $R^4$ die angegebene Bedeutung haben, hydrogenolytisch oder durch Behandlung mit Mineralsäure zu Verbindungen der Formel II debenzyliert.

9. Verbindungen der Formel VI, wie im Anspruch 8 definiert, mit der Maßgabe der Rest $R^3$ mindestens 2 C-Atome enthält.

10. Verwendung von Verbindungen der Formel I aus Anspruch 1 zur Herstellung von Pflanzenschutzmitteln auf der Basis von Pyrimidinylamino-oder Pyridinylamino-Heterocyclen.

11. Verfahren zur Herstellung von Pflanzenschutzmitteln auf der Basis von Pyrimidinylamino-oder Pyridinylamino-Heterocyclen, dadurch gekennzeichnet, daß die Verbindungen der Formel I aus Anspruch 1 als Ausgangsstoffe verwendet werden.

Europäisches Patentamt

# EUROPÄISCHER RECHERCHENBERICHT

Nummer der Anmeldung: EP 87119194.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US - A - 3 784 608 (E.R.LARSEN et al.)<br>* Spalte 2, Zeilen 10-21; Beispiele 9,10; Spalte 9, Zeilen 12-34; Spalte 9, Zeile 58 - Spalte 10, Zeile 2 *<br>-- | 1,3,4,6,8,10,11 | C 07 C 79/35<br>C 07 C 79/46<br>C 07 C 121/75<br>C 07 C 76/02<br>C 07 C 43/225<br>C 07 C 41/00<br>C 07 C 69/92<br>A 01 N 43/40<br>A 01 N 43/54 |
| A | DE - A1 - 3 402 483 (MISUI TOATSU)<br>* Verbindung Nr. 48 *<br>-- | 9 | |
| A | GB - A - 2 097 000 (RIKER LABORATORIES)<br>* Seite 1, Zeilen 1-15 *<br>-- | 8 | |
| A | US - A - 4 360 700 (MELVIN, JR.)<br>* Spalte 2, Zeilen 37-46; Anspruch 1 *<br>-- | 8,9 | **RECHERCHIERTE SACHGEBIETE (Int. Cl.4)** |
| A | HOUBEN WEYL "Methoden der organischen Chemie", 4. Auflage, Band VI/1c, Teil 1 "Phenole", 1976<br>GEORG THIEME VERLAG, Stuttgart Seiten 158-166<br>* Seite 164, Zeile 25 - Seite 166, Zeile 25 *<br>---- | 7 | C 07 C 79/00<br>C 07 C 121/00<br>C 07 C 76/00<br>C 07 C 43/00<br>C 07 C 41/00<br>C 07 C 69/00 |

Der vorliegende Recherchenbericht wurde für alle Patentansprüche erstellt.

| Recherchenort | Abschlußdatum der Recherche | Prüfer |
|---|---|---|
| WIEN | 08-04-1988 | KÖRBER |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82